(19) Europäisches Patentamt
European Patent Office
Office européen des brevets

(11) Numéro de publication : **0 328 434 B1**

(12) # FASCICULE DE BREVET EUROPEEN

(45) Date de publication du fascicule du brevet : **31.03.93 Bulletin 93/13**

(51) Int. Cl.$^5$ : **C07C 221/00,** C07C 225/16, C07C 233/33, C07C 233/42, A61K 31/135, A61K 31/16

(21) Numéro de dépôt : **89400248.4**

(22) Date de dépôt : **30.01.89**

(54) **Dérivés de 1-(aminophényl)-2-diméthylaminopropanone, procédé de préparation et utilisation en thérapeutique.**

(30) Priorité : **10.02.88 FR 8801564**

(43) Date de publication de la demande : **16.08.89 Bulletin 89/33**

(45) Mention de la délivrance du brevet : **31.03.93 Bulletin 93/13**

(84) Etats contractants désignés : **AT BE CH DE ES FR GB GR IT LI LU NL SE**

(56) Documents cités :
**EP-A- 0 138 714**
**EP-A- 0 174 242**

(73) Titulaire : **LABORATOIRE L. LAFON**
**19 Avenue du Professeur Cadiot**
**F-94701 Maisons Alfort (FR)**

(72) Inventeur : **Lafon, Louis**
**5, rue de l'Alboni**
**F-75016 Paris (FR)**

(74) Mandataire : **Clisci, Serge et al**
**S.A. FEDIT-LORIOT CONSEILS EN PROPRIETE INDUSTRIELLE 38, avenue Hoche**
**F-75008 Paris (FR)**

EP 0 328 434 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne en tant que produits industriels nouveaux des dérivés de 1-(aminophényl)-2-diméthylaminopropanone. Elle concerne également le procédé de préparation de ces produits et leur utilisation en thérapeutique.

## ART ANTERIEUR

On sait que l'on a déjà préconisé dans le passé des dérivés de 1-(aminophényl)-2-aminopropanone de formule

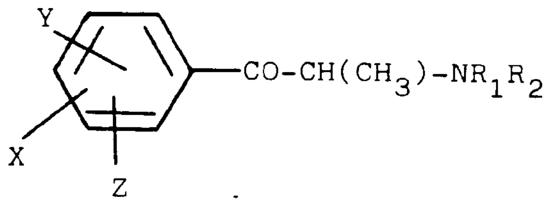

$(I_o)$

dans laquelle
X est $NH_2$ ou $CH_3CONH$
Y est un atome d'hydrogène ou d'halogène,
Z est un atome d'hydrogène ou d'halogène,
$R_1$ est un groupe alkyle en $C_1$-$C_4$ ou un groupe cycloalkyle en $C_3$-$C_6$,
$R_2$ est l'atome d'hydrogène ou un groupe alkyle en $C_1$-$C_4$,
$R_1$ et $R_2$, considérés ensemble, peuvent former avec l'atome d'azote auquel ils sont liés un groupe N-hétérocyclique de 5 à 7 sommets, pouvant comporter un second hétéroatome choisi parmi N,O et S et pouvant être substitué, ledit groupe hétérocyclique $NR_1R_2$ étant choisi parmi l'ensemble comprenant les groupes pyrrolidino, morpholino, thiomorpholino, pipéridino, hexaméthylèneimino, pipérazino, 4-méthylpipérazino, 4-(2-hydroxyéthyl)-pipérazino, 4-phénylpipérazino, 4-(p-chlorophényl)-pipérazino ;
et leurs sels d'addition, en tant qu'agents antidépresseurs du système nerveux central (SNC). On sait également que, parmi les composés de formule $I_O$ ci-dessus, quelques produits seulement présentent en outre des propriétés cardiovasculaires et/ou immunologiques bénéfiques. Voir à cet effet le brevet EP-B-0 174 242 relatif aux composés aminés ($X=NH_2$) et le brevet EP-B-0 138 714 relatif aux composés acétylaminés ($X=CH_3CONH$).

## BUT ET OBJET DE L'INVENTION

Selon l'invention on propose de nouveaux produits appartenant à la famille des dérivés de 1-(aminophényl)-2-aminopropanone et fournit leur procédé de préparation, ces nouveaux produits étant particulièrement utiles en thérapeutique.

Ces nouveaux produits, qui sont inclus dans les définitions générales de la formule $I_o$ mais qui n'ont pas encore été spécifiquement décrits jusqu'à ce jour, possèdent des propriétés antidépressives comme les composés de formule $I_o$ décrits dans EP-B-0 174 242 et EP-B-0 138 714 précités. Ils se distinguent de ces composés antérieurement décrits par le fait qu'ils exercent des effets sédatifs alors que les composés décrits dans EP-B-0 174 242 et EP-B-0 138 714 présentent généralement des effets psychostimulants et/ou éveillants.

Selon un autre aspect de l'invention on propose des composés, à savoir la 1-(4-acétylamino-3,5-dichlorophényl)-2-diméthylaminopropanone et ses sels d'addition, qui sont doués de propriétés immunologiques bénéfiques alors que leurs homologues structurellement voisins sont dépourvus desdites propriétés immunologiques.

Les composés selon l'invention sont caractérisés en ce qu'ils sont choisis parmi l'ensemble comprenant les composés répondant à la formule générale

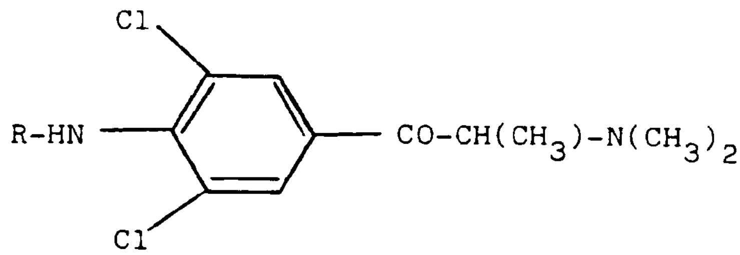

(I)

où R est H ou $CH_3CO$, et leurs sels d'addition.

## DESCRIPTION DETAILLEE DE L'INVENTION

L'invention concerne donc la 1-(4-amino-3,5-dichlorophényl)-2-diméthylaminopropanone, la 1-(4-acétylamino-3,5-dichlorophényl)-2-diméthylaminopropanone et leurs sels d'addition.

Par sels d'addition, on entend ici les sels d'addition d'acide obtenus par réaction d'une base libre de formule I avec un acide minéral ou organique, d'une part, et les sels d'ammonium, d'autre part. Parmi les acides utilisables pour salifier la base libre de formule I, on peut notamment mentionner les acides chlorhydrique, bromhydrique, acétique, formique, propionique, oxalique, fumarique, maléique, succinique, benzoïque, cinnamique, mandélique, citrique, malique, tartrique, aspartique, glutamique, méthanesulfonique, p-toluènesulfonique. Parmi les composés permettant d'obtenir des sels d'ammonium, on peut notamment citer $ICH_3$ et $ClCH_3$. D'une manière générale les sels d'addition d'acide, tels que notamment les chlorhydrates, sont préférés aux sels d'ammonium.

Un certain nombre de composés selon l'invention a été consigné de façon nullement limitative dans le tableau I ci-après, avec, pour comparaison, les deux homologues CP-1 et CP-2 décrits dans les documents précités.

**TABLEAU I**

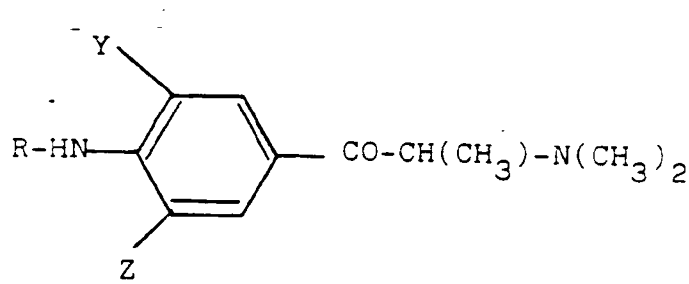

| Exemple | No de Code | R | Y | Z |
|---|---|---|---|---|
| Ex 1 (a) | CRL 41 403 | $CH_3CO$ | 3-Cl | 5-Cl |
| Ex 2 (a) | CRL 41 402 | H | 3-Cl | 5-Cl |
| Ex 3 (b) | - | $CH_3CO$ | 3-Cl | 5-Cl |
| CP-1(c,d) | CRL 41 233 | H | H | H |
| CP-2(a,e) | CRL 41 232 | $CH_3CO$ | H | H |

Notes

(a) : monochlorhydrate ;

(b) : méthanesulfonate ;

(c) : dichlorhydrate ;

(d) : décrit à l'exemple 9 de EP-B-0 174 242 ;

(e) : décrit à l'exemple 9 de EP-B-0 138 714.

Les composés de formule I peuvent être préparés selon une méthode connue en soi par application de mécanismes réactionnels classiques.

En particulier ils peuvent être synthétisés suivant les méthodes opératoires décrites dans EP-B-0 174 242 et EP-B-0 138 714 précités.

Le procédé que l'on préconise ici consiste :

1°) à faire réagir 1 mole de 1-(4-aminophényl)-2-diméthylaminopropanone avec au moins 2 moles de N-chlorosuccinimide, à une température comprise entre 5 et 20°C pendant au moins 4 h, pour obtenir le composé de formule le I où R = H ; et,

2°) si nécessaire, à soumettre ledit composé de formule I où R = H, à une réaction d'acétylation, pour obtenir le composé de formule I où R = $CH_3CO$.

Lors de la mise en oeuvre du stade 1°) on opère avec un excès de N-chlorosuccinimide par rapport aux conditions stoechiométriques ; la réaction est réalisée avantageusement à 10°C lors de l'introduction par fractions du chlorosuccinimide dans une solution de 1-(4-aminophényl)-2-diméthylaminopropanone dans un solvant approprié, puis poursuivie à 15-20°C pendant 8-12 h.

Au stade 2°) on traite à la température ambiante (15-20°C) 1 mole de 1-(4-amino-3,5-dichlorophényl)-2-diméthylaminopropanone avec au moins 3 moles de chlorure d'acétyle dans de l'acide acétique pendant 8-12 h.

Les composés selon l'invention présentant des propriétés thérapeutiques bénéfiques. Ils agissent en particulier en tant que moyens antidépresseurs du SNC et possèdent des effets sédatifs inattendus eu égard aux effets psychostimulants et/ou éveillants des composés homologues CP-1 et CP-2.

Par ailleurs la 1-(4-acétylamino-3,5-dichlorophényl)-2-diméthylaminopropanone et ses sels d'addition non-toxiques, sont particulièrement intéressants en raison de leurs propriétés immunologiques, en ce sens qu'ils agissent en tant qu'agents immunostimulants notamment vis-à-vis de l'immunité à médiation cellulaire et vis-à-vis de l'immunité à médiation humorale.

Selon l'invention on préconise une composition thérapeutique qui est caractérisée en ce qu'elle renferme, en association avec un excipient physiologiquement acceptable, au moins un dérivé choisi parmi les composés de formule I et leurs sels d'addition non-toxiques.

Bien entendu, dans une telle composition le principe actif, à savoir le composé de formule I ou l'un de ses sels non-toxiques, intervient en quantité pharmaceutiquement efficace.

Selon l'invention, on préconise l'utilisation d'une substance choisie parmi l'ensemble comprenant : (i) la 1-(4-acétylamino-3,5-dichlorophényl)-2-diméthylaminopropanone, (ii) la 1-(4-amino-3,5-dichlorophényl)-2-diméthylaminopropanone et (iii) leurs sels d'addition non-toxiques, pour l'obtention d'un médicament antidépresseur du SNC destiné à une utilisation en thérapeutique humaine vis-à-vis des dépressions et des états dépressifs.

Selon l'invention, on préconise également l'utilisation d'une substance choisie parmi l'ensemble comprenant la 1-(4-acétylamino-3,5-dichlorophényl)-2-diméthylaminopropanone et ses sels d'addition non toxiques, pour l'obtention d'un médicament immuno-stimulant destiné à une utilisation en thérapeutique humaine dans le cas où une immuno-stimulation est requise.

D'autres avantages et caractéristiques de l'invention seront mieux compris à la lecture qui va suivre d'exemples de préparation et de résultats d'essais pharmacologiques, l'ensemble de ces éléments n'étant nullement limitatif mais donné à titre d'illustration.

**PREPARATION I**

Obtention du monochlorhydrate de 1-(4-acétylamino-3,5-dichlorophényl)-2-diméthylamino-propanone

(Exemple 1 ; No de Code : CRL 41 403)

On agite pendant une nuit, un mélange de 16 g (0,053 mole) de chlorhydrate de 1-(4-amino-3,5-dichlorophényl)-2-diméthylaminopropanone (No de Code : CRL 41 402) et de 11,5 ml (0,160 mole) de chlorure d'acétyle dans 75 ml d'acide acétique. On évapore à sec le mélange réactionnel sous pression réduite et reprend le résidu d'évaporation par de l'acétone. Par filtration on recueille 16,6 g (rendement : 92,25 %) de CRL 41 403 sous la forme d'une poudre de couleur beige. F = environ 200°C (avec décomposition).

PREPARATION II

Obtention du monochlorhydrate de 1-(4-amino-3,5-dichlorophényl)-2-diméthylaminopropanone

(Exemple 2 ; No de Code : CRL 41 402)

Au sein d'une solution maintenue à environ 10°C de 22,5 g (0,083 mole) de dichlorohydrate de 1-(4-aminophényl)-2-diméthylaminopropanone (No de Code : CRL 41 233 ; référencé ici : CP-1) dans 100 ml d'eau, on introduit par fraction en 1 heure 26,7 g (0,200 mole) de N-chlorosuccinimide, puis laisse le milieu réactionnel pendant 1 nuit à la température ambiante (15-20°C). On recueille par filtration le précipité formé, lave ledit précipité avec de l'acétone à chaud. On obtient 17 g (rendement : 68,85 %) de CRL 41 402 sous la forme d'une poudre beige soluble dans l'eau à 50 g/l.$F_{inst}$ > 260°C.

On a résumé ci-après les résultats des essais toxicologiques, neuropsychopharmacologiques et immunologiques qui ont été entrepris avec les composés selon l'invention.

A. ESSAIS RELATIFS AU CRL 41 403 (PRODUIT DE L'EXEMPLE 1)

- ETUDE NEUROPSYCHOPHARMACOLOGIQUE

Dans l'étude neuropsychopharmacologique qui suit, le CRL 41 403 en solution dans de l'eau distillée (solution à pH de 5,5) a été administré par voie intrapéritonéale sous un volume de 20 ml/kg chez la souris mâle et de 5 ml/kg chez le rat mâle.

## I. TOXICITE

Chez la souris mâle la DL-0 (dose maximale non mortelle) par voie intrapéritonéale est supérieure à 128 mg/kg et la DL-60 (dose létale pour 60 % des animaux traités) est de l'ordre de 256 mg/kg environ (à cette dose la mort des souris intervient dans les 24 heures qui suivent l'administration I.P. du CRL 41 403). La DL-100 (dose minimale mortelle pour tous les animaux traités) est inférieure à 512 mg/kg (à cette dose la mort des souris intervient dans les 8-10 minutes qui suivent l'administration du CRL 41 403).

## II. COMPORTEMENT GLOBAL ET REACTIVITES

Des lots de trois animaux sont observés avant, puis 0,25 h, 0,50 h, 1 h, 2 h, 3 h et 24 h après administration de CRL 41 403. On constate :

1°) chez la souris

aux doses de 1 mg/kg, 4 mg/kg et 16 mg/kg :
- un comportement et des réactivités sensiblement comparables à ceux du lot témoin

à la dose de 64 mg/kg :
- une diminution de la fréquence respiratoire avec une mydriase pendant 2 heures ;

à la dose de 128 mg/kg :
- une sédation ;
- un effet hypothermisant très modéré, et
- une diminution de la fréquence respiratoire; et,

2°) chez le rat

aux doses de 0,25 mg/kg, 2 mg/kg et 8 mg/kg :
- un comportement, des réactivités, une variation de la température rectale et du diamètre pupillaire sensiblement comparables à ceux du lot témoin ;

à la dose de 32 mg/kg :
- une mydriase pendant 1 h.

## III. INTERACTION AVEC L'APOMORPHINE

1°) Chez la souris

Des lots de 6 souris reçoivent, par voie I.P., le produit à tester, 0,5 h avant l'injection sous-cutanée de 1 ou 16 mg/kg d'apomorphine. On observe que, dès la dose de 1 mg/kg et surtout à la dose de 64 mg/kg, le CRL 41 403 s'oppose à l'hypothermie induite par l'apomorphine sans modifier les comportements de verticalisation et les stéréotypies.

Il convient de noter que, à la plus forte dose utilisée (64 mg/kg), l'effet hypothermisant très modéré du CRL 41 403 se manifeste avant l'injection d'apomorphine.

2°) Chez le rat

Le produit à tester est administré à des lots de 6 rats 0,5 h avant l'injection sous-cutanée de 0,5 mg/kg d'apomorphine. On observe que, dès la dose de 0,5 mg/kg le CRL 41 403 ne modifie pas les stéréotypies induites par l'apomorphine.

## IV. INTERACTION AVEC L'AMPHETAMINE

L'amphétamine (2 mg/kg) est injectée par voie intrapéritonéale à des lots de 6 rats, 30 minutes après l'administration du produit à tester. On constate que, aux doses de 1 mg/kg à 64 mg/kg, le CRL 41 403 ne modifie pas les stéréotypies amphétaminiques.

## V. INTERACTION AVEC LA RESERPINE

Quatre heures après l'injection intrapéritonéale de 2,5 mg/kg de réserpine, des lots de 6 souris reçoivent le produit à tester.

On note que, dès la dose de 1 mg/kg et surtout aux doses de 16 mg/kg et de 64 mg/kg, le CRL 41 403 s'oppose à l'hypothermie réserpinique [ aux doses inférieures (1 et 4 mg/kg) l'effet antihypothermisant est fugace et ne se manifeste qu'une heure après l'administration du CRL 41 403 ]. On observe par ailleurs que le

CRL 41 403, à la dose de 16 mg/kg et surtout à la dose de 64 mg/kg, diminue le ptôsis réserpinique.

## VI. INTERACTION AVEC L'OXOTREMORINE

Le produit à étudier est administré à des lots de 6 souris 0,5 h avant l'injection intrapéritonéale de 0,5 mg/kg d'oxotrémorine.

1°) Action sur la température

Dès la dose de 4 mg/kg et surtout à la dose de 64 mg/kg, le CRL 41 403 s'oppose à l'hypothermie induite par l'oxotrémorine. Il convient de noter que, à la plus forte dose utilisée (64 mg/kg), l'effet hypothermisant du CRL 41 403 se manifeste avant l'administration de l'oxotrémorine.

2°) Action sur les tremblements

On constate que, à la plus forte dose utilisée (64 mg/kg), le CRL 41 403 diminue modérément l'intensité des tremblements induits par l'oxotrémorine.

3°) Action sur les symptômes cholinergiques périphériques

On observe que le CRL 41 403 ne modifie pratiquement pas les signes de stimulation cholinergiques périphériques dûs à l'oxotrémorine.

## VII. ACTION SUR LE TEST DES QUATRE PLAQUES, LA TRACTION ET L'ELECTROCHOC

Le test est pratiqué sur des lots de 10 souris, 30 minutes après l'administration du produit à étudier.

On constate que le CRL 41 403 ne modifie pratiquement pas le nombre de passages punis, ne provoque pas d'incapacité motrice majeure, et, ne modifie pas les effets convulsivants et létaux de l'électrochoc.

## VIII. ACTION SUR LA MOTILITE SPONTANEE

0,5 h après avoir reçu le produit à étudier, les souris (12 par dose, 24 témoins) sont placées en actimètre où leur motilité est enregistrée pendant 30 minutes.

On constate que, aux deux plus fortes doses utilisées (16 et 64 mg/kg), le CRL 41 403 diminue modérément l'activité motrice spontanée de la souris.

## IX. ACTION SUR L'AGRESSIVITE INTERGROUPES

Après avoir séjourné pendant 3 semaines dans chacune des moitiés d'une cage séparées par une cloison opaque, des groupes de 3 souris reçoivent le produit à étudier. Une demi-heure plus tard, les deux groupes d'une même cage sont réunis par retrait de la cloison et on note le nombre de combats qui surviennent en 10 minutes.

On constate que, à la dose de 16 mg/kg et surtout à la dose de 64 mg/kg, le CRL 41 403 diminue nettement le nombre de combats.

## X. ACTION VIS-A-VIS DE QUELQUES COMPORTEMENTS PERTURBES PAR DIVERS AGENTS

1°) Motilité réduite par habituation à l'enceinte

Après 18 heures de séjour dans les actimètres, les souris (6 par dose, 12 témoins) reçoivent le produit à tester. Elles sont aussitôt replacées dans leurs enceintes respectives et, une demi-heure plus tard on enregistre leur motilité pendant 30 minutes.

On observe que le CRL 41 403 n'entraîne pas de reprise de l'activité motrice chez la souris habituée à son enceinte.

2°) Motilité réduite par agression hypoxique

Une demi-heure après avoir reçu le produit à tester, les souris (10 par dose, 20 témoins) sont soumises à

une anoxie hypobare aigüe [ dépression de 600 mmHg (i.e. environ 8 x $10^4$Pa) en 90 secondes ; détente de 45 secondes ], puis elles sont placées en actimètre où leur motilité est enregistrée pendant 10 minutes.

On observe que, à la dose de 64 mg/kg, le CRL 41 403 entraîne une nette amélioration de la récupération motrice chez la souris dont la motilité a été déprimée à la suite d'un séjour bref dans une enceinte à pression réduite.

3°) Anoxie asphyxique

Des lots de 10 souris reçoivent le produit à tester une demi-heure avant l'administration intrapéritonéale de 32 mg/kg de triiodoéthylate de gallamine (agent curarisant de référence).

On observe que le CRL 41 403 ne modifie pratiquement pas le délai d'apparition des convulsions et de la mort consécutives à une anoxie asphyxique provoquée par un agent curarisant.

**XI. INTERACTION AVEC LE BARBITAL**

Une demi-heure après l'administration du produit à étudier, des lots de 10 souris reçoivent une injection intrapéritonéale de barbital (220 mg/kg).

On remarque que, à la plus forte dose utilisée (64 mg/kg), le CRL 41 403 diminue nettement la durée du sommeil barbiturique.

**XII. ACTION SUR LE "DESESPOIR COMPORTEMENTAL"**

Une demi-heure après avoir reçu le produit à tester, des lots de 6 souris sont placés dans un bécher rempli d'eau sur une hauteur de 6 cm. On note la durée totale d'immobilité entre la deuxième et la sixième minutes suivant l'immersion.

On observe que, à la plus forte dose étudiée (64 mg/kg), le CRL 41 403 diminue la durée d'immobilité de la souris placée en immersion forcée.

**XIII. CONCLUSIONS**

Il résulte de l'ensemble des essais neuropsychopharmacologiques ci-dessus que le CRL 41 403 présente des effets

- antidépresseurs objectivés par l'antagonisme des hypothermies induites par l'apomorphine, la réserpine ou l'oxotrémorine, et, par la diminution à forte dose de la durée de l'immobilité de "désespoir" ; et,
- sédatifs qui ne se manifestent qu'à forte dose par une diminution modérée de l'activité motrice spontanée chez la souris, une diminution de l'agressivité intergroupes chez la souris, et, une hypothermie chez la souris.

Ces effets sédatifs permettent de distinguer le CRL 41 403 de ses homologues CP-1 et CP-2 précités qui exercent des effets éveillants et stimulants à forte dose.

A côté de ces effets sédatifs, le CRL 41 403 présente de façon ponctuelle dans son profil neuropsychopharmacologique des activités éveillante et antihypoxique. L'activité éveillante est objectivée par une diminution de la durée du sommeil barbiturique. L'activité antihypoxique (qui n'est pas du à un effet stimulant et/ou anticonvulsivant) est objectivée par une nette amélioration de la récupération motrice après hypoxie hypobare aigüe.

Ces activités ponctuelles éveillante et antihypoxique sont paradoxalement surprenantes, car à la plus forte dose utilisée (64 mg/kg) le CRL 41 403 présente, comme illustré ci-dessus, des effets sédatifs qui sont manifestement contraires auxdites activités éveillante et antihypoxique.

- ESSAIS COMPLEMENTAIRES

Des essais complémentaires ont été entrepris par voie gastrique chez le rat pour apprécier une éventuelle toxicité suivant un tel mode d'administration.

Le CRL 41 403, en solution dans de l'eau distillée pour des concentrations inférieures ou égales à 6,4 g/l ou en suspension dans une solution aqueuse de gomme arabique pour des concentrations supérieures ou égales à 12,4 g/l, a été administré par voie gastrique, au moyen d'une sonde appropriée, chez le rat mâle sous un volume de 5 ml/kg.

Des lots de 3 rats sont observés avant, puis 0,25 h, 0,50 h, 1 h, 2 h, 3 h et 24 h après l'administration de CRL 41 403 par voie gastrique. On constate :

aux doses de 2 mg/kg, 8 mg/kg, 32 mg/kg et 128 mg/kg:

- un comportement, des réactivités, une variation de la température rectale et du diamètre pupillaire sensiblement comparables à ceux du lot témoin,

aux doses de 256 mg/kg et 512 mg/kg :

- aucun symptome particulier, et

à la dose de 1 024 mg/kg :

- une sédation,
- une augmentation de la salivation,
- une diminution de la fréquence respiratoire,
- aucune mortalité,

On observe donc dans les présentes conditions expérimentales que le CRL 41 403, administré par voie gastrique chez le rat n'est pas toxique et ne montre des modifications nettes du comportement qu'à la plus forte dose utilisée. L'augmentation de la salivation semble être en relation avec une composante de stimulation alpha-adrénergique déjà observée après administration I.P. (mydriase chez la souris, diminution du ptôsis réserpinique).

- ETUDE IMMUNOLOGIQUE

Les propriétés d'agent immuno-modulateur du CRL 41 403 ont été étudiées suivant plusieurs protocoles par comparaison avec le CRL 41 402 (produit de l'exemple 2), le CRL 41 233 décrit dans EP-B-0 174 242 (et désigné ici CP-1) et le CRL 41 232 décrit dans EP-B-0 138 714 (et désigné ici CP-2).

On a notamment utilisé le test dit des cellules formant des plages de lyse décrit par A.J. CUNNINGHAM et al. ("Further improvements in the plaque technique for detecting simple antibody forming cells"), Immunology 14, pages 599-601, (1968), d'une part, et le test dit de l'intensité de l'hypersensibilité retardée aux globules rouges de mouton décrit par T.E. MILLER et al. ("Immunopotentiation with BCG II modulation of the response to sheep blood cells"), Journal of the National Cancer Institute 51 (N° 5), pages 1669-1676, (1973), d'autre part.

1°) Le test des cellules formant plages de lyse (ou PFC IgM) explore l'immunité humorale. Quatre jours après immunisation par un antigène T dépendant (ici des globules rouges de mouton), on dénombre les cellules spléniques exprimant une réponse directe anticorps IgM. Les souris utilisées à cet effet sont des souris conventionnelles $OF_1$ femelles, exemptes d'organismes pathogènes spécifiques, pesant de 20 à 30 g, et, réparties suivant un lot témoin de 14 animaux et de lots de 7 animaux chacun par dose et par produit à étudier administré par voie orale le même jour que l'antigène. Un indice d'activité I est calculé pour chaque dose de produit selon la relation :

$$I = \frac{\text{moyenne de lyses par rate de souris traitées}}{\text{moyenne de lyses par rate des souris témoins}}$$

Ce test est réalisé au moins deux fois pour chaque dose (0,001 ; 0,01 ; 0,1 ; 10 ; et 100 mg/kg) de produit à tester, et une étude statistique est entreprise au moyen du test t de Student après analyse de variance sur le nombre de lyses par rate.

On constate que l'indice d'activité augmente à toutes les doses pour le CRL 41 403 pour atteindre la valeur I = 2 à la dose de 100 mg/kg P.O., alors que les trois autres produits, EX 2, CP-1 et CP-2, ne modifient pas ledit indice d'activité.

2°) Le test de l'intensité de l'hypersensibilité retardée aux globules rouges de mouton est une technique d'exploration de l'immunité cellulaire. Le composé à étudier est administré soit per os, s'il est insoluble, soit (comme c'est le cas ici) par voie sous-cutanée dans le coussinet de la patte, lorsqu'il est soluble dans l'eau, l'administration dudit composé intervenant trois jours avant immunisation (par administration sous-cutanée de globules rouges de mouton dans le coussinet plantaire) de souris conventionnelles $OF_1$ femelles réparties suivant un lot témoin de 10 animaux et des lots de 5 animaux chacun par dose et par produit à étudier. Les résultats obtenus sont exprimés en pourcentage d'augmentation de l'épaisseur du coussinet plantaire.

Ce test est réalisé au moins deux fois pour chaque dose (0,001 ; 0,01 ; 0,1 ; 10 ; et 100 mg/kg) de produit à tester et une étude statistique est entreprise comme indiqué ci-dessus.

On constate que le CRL 41 403 est le seul produit testé qui donne une réaction positive d'hypersensibilité retardée. A la dose de 100 mg/kg S.C., le CRL 41 403 induit une variation en pourcentage d'augmentation de $16{,}40 \pm 4{,}06$ de l'épaisseur du coussinet plantaire, alors que les témoins donnent une variation en pourcentage de $8{,}28 \pm 1{,}14$;

Tous ces résultats notamment les résultats consignés dans le tableau II ci-après, mettent en évidence que le CRL 41 403 (i) est un produit immunomodulateur à la différence des trois autres produits, et (ii) agit plus précisément en tant que substance immuno-stimulante.

## TABLEAU II

## ETUDE IMMUNOLOGIQUE

## ESSAIS COMPARATIFS

| Produit | No de Code | dose (a) | I(b) | HSR (c) |
|---|---|---|---|---|
| Ex 1 | CRL 41 403 | 100 | 2,00* | 16,40* |
| Ex 2 | CRL 41 402 | 10 | 1,09 | 8,26 |
| CP-1 | CRL 41 233 | 100 | 0,98 | 8,29 |
| CP-2 | CRL 41 232 | 100 | 1,01 | 8,27 |
| témoins | - | - | 1 | 8,28 |

Notes

(a) dose exprimée en mg/kg, le mode d'administration étant la voie orale pour la mesure de l'indice d'activité I, et la voie sous-cutanée pour la mesure de l'hypersensibilité retardée HSR ;

(b) indice d'activité suivant le test dit des cellules formant des plages de lyse

$$I = \frac{\text{moyenne de lyses par rate des souris traitées}}{\text{moyenne de lyses par rate des souris témoins}}$$

(c) intensité de l'hypersensibilité retardée aux globules rouges de mouton exprimée en pourcentage d'augmentation de l'épaisseur du coussinet plantaire ;

* résultat statistiquement significatif par rapport aux témoins.

B. ESSAIS RELATIFS AU CRL 41 402 (PRODUIT DE L'EXEMPLE 2)

L'étude neuropsychopharmacologique du CRL 41 402 a été entreprise selon les modalités opératoires décrites ci-dessus pour le CRL 41 403, le CRL 41 402 à étudier étant administré en solution dans de l'eau distillée pour des concentrations inférieures à 25 g/l, ou, en suspension dans une solution aqueuse de gomme arabique pour des concentrations supérieures ou égales à 25 g/l, par voie intrapéritonéale, sous un volume de 20 ml/kg chez la souris mâle et de 5 ml/kg chez le rat mâle.

Le pH de la composition administrée varie modérément en fonction de la concentration en CRL 41 402 : il passe de 6,0 pour une concentration de 125 g/l à 5,5 pour les concentrations inférieures ou égales à 6,4 g/l.

### I. TOXICITE

La DL-O du CRL 41 402 est supérieure à 32 mg/kg. La DL-30 du CRL 41 402 est par voie intrapéritonéale

de l'ordre de 64 mg/kg.

## II. COMPORTEMENT GLOBAL ET REACTIVITES

1°) chez la souris

aux doses de 0,25 mg/kg, 1 mg/kg et 4 mg/kg :

- un comportement et des réactivités sensiblement comparables à ceux du lot témoin ;

à la dose de 16 mg/kg :

- une salivation se manifestant pendant 0,5 h environ, entre la demi-heure et l'heure qui suivent l'administratration du CRL 41 402 ;
- une sédation ;
- une diminution de la fréquence respiratoire, et
- une hypothermie modérée pendant 3 h (variation de -1,4°C 1 h après administration du CRL 41 402 ; alors que dans les mêmes conditions opératoires la variation de la température des témoins est de - 0,9°C) ;

à la dose de 32 mg/kg :

une sédation,
- une diminution de la fréquence respiratoire, et
- une salivation ; et,

2°) chez le rat

aux doses de 0,125 mg/kg, 0,5 mg/kg et 2 mg/kg :

- un comportement, des réactivités, une variation de la température rectale et du diamètre pupillaire sensiblement comparables à ceux du lot témoin ; et,

à la dose de 8 mg/kg :

- une mydriase modérée pendant 2 h.

## III. INTERACTION AVEC L'APOMORPHINE

Chez la souris, aux doses de 0,25 mg/kg, de 1 mg/kg et surtout aux doses de 4 mg/kg et de 16 mg/kg, le CRL 41 402 s'oppose à l'hypothermie induite par l'apomorphine, mais ne modifie pas les comportements de verticalisation.

Chez le rat, on constate que le CRL 41 402 ne modifie pas les stéréotypies induites par l'apomorphine.

## IV. INTERACTION AVEC L'AMPHETAMINE

On observe que le CRL 41 402 ne modifie pratiquement pas les stéréotypies provoquées par l'amphétamine.

## V. INTERACTION AVEC LA RESERPINE

Aux doses de 1 mg/kg, 4 mg/kg et 16 mg/kg , le CRL 41 402 antagonise nettement l'hypothermie induite par la réserpine.

## VI. INTERACTION AVEC L'OXOTREMORINE

Aux doses de 4 et 16 mg/kg, le CRL 41 402 s'oppose à l'action hypothermisante de l'oxotrémorine.

Le CRL 41 402 ne modifie pas les tremblements provoqués par l'oxotrémorine.

Enfin, le CRL 41 402 ne semble pas modifier de façon nette les signes de stimulation cholinergique périphérique dus à l'oxotrémorine.

## VII. ACTION SUR LE TEST DES QUATRE PLAQUES, LA TRACTION ET L'ELECTROCHOC

On observe que le CRL 41 402 ne modifie pas le nombre de passages punis, qu'il ne provoque pas d'incapacité motrice majeure, et qu'il ne modifie pas les effets convulsivants et létaux de l'électrochoc.

## VIII. ACTION SUR LA MOTILITE SPONTANEE

On constate que le CRL 41 402 entraîne aux deux plus fortes doses utilisées (4 et 16 mg/kg) une diminution

modérée de la motilité spontanée de la souris.

### IX. ACTION SUR L'AGRESSIVITE INTERGROUPES

On observe que dès la dose de 1 mg/kg le CRL 41 402 diminue nettement le nombre de combats.

### X. ACTION VIS-A-VIS DE QUELQUES COMPORTEMENTS PERTURBES PAR DIVERS AGENTS

1°) Motilité réduite par habituation à l'enceinte

On constate que le CRL 41 402 n'entraîne pas de reprise nette de l'activité motrice chez la souris habituée à son enceinte.

2°) Motilité réduite par agression hypoxique

A la dose de 4 mg/kg et surtout à la dose de 16 mg/kg, le CRL 41 402 entraîne une nette amélioration de la récupération motrice chez la souris dont la motilité a été déprimée à la suite d'un séjour bref dans une enceinte à pression réduite.

3°) Anoxie asphyxique

Le CRL 41 402 ne modifie pratiquement pas le délai d'apparition des convulsions et de la mort consécutives à une anoxie asphyxique provoquée par un agent curarisant.

### XI. INTERACTION AVEC LE BARBITAL

On constate que le CRL 41 402 ne modifie pas la durée du sommeil barbiturique.

### XII. ACTION SUR LE "DESESPOIR COMPORTEMENTAL"

On observe que, à la plus forte dose utilisée (16 mg/kg), le CRL 41 402, diminue nettement la durée d'immobilité de la souris placée en immersion forcée.

### XIII. CONCLUSIONS

L'ensemble des essais neuropsychopharmacologiques met en évidence que le CRL 41 402 présente des effets

- antidépresseurs objectivés par l'antagonisme des hypothermies induites par l'apomorphine, l'oxotrémorine ou la réserpine, et, par la diminution de l'immobilité dite "de désespoir" ; et,
- sédatifs modérés qui se manifestent par une diminution (faible) de l'activité motrice en association avec une diminution de l'agressivité, d'une part, et une hypothermie modeste, d'autre part.

En clinique on a obtenu de bons résultats en administrant chez l'homme adulte le CRL 41 403 en tant qu'agent antidépresseur, d'une part, et en tant que substance immuno-stimulante, d'autre part. En particulier, le CRL 41 403 s'est avéré être un excellent antidépresseur à la dose quotidienne de 15 mg (répartie en trois prises de chacune 5 mg), notamment chez des brûlés souffrant de dépression.

## Revendications

### Revendications pour les Etats contractants suivants : AT, BE, CH, DE, FR, GB, IT, LI, LU, NL, SE

**1.** Dérivé de 1-(4-aminophényl)-2-diméthylaminopropanone, caractérisé en ce qu'il est choisi parmi l'ensemble constitué par:

(a) les composés répondant à la formule générale

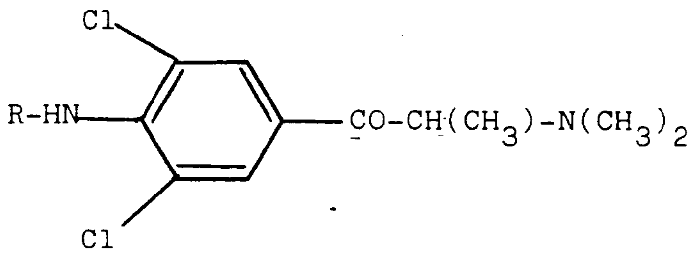

(I)

où R est H ou $CH_3CO$, et
(b) leurs sels d'addition.

2. 1-(4-Acétylamino-3,5-dichlorophényl)-2-diméthylaminopropanone et ses sels d'addition.

3. 1-(4-Amino-3,5-dichlorophényl)-2-diméthylaminopropanone et ses sels d'addition.

4. Composition thérapeutique caractérisée en ce qu'elle renferme, en association avec un excipient physiologiquement acceptable, au moins un composé choisi parmi les composés de formule I suivant la revendication 1 et leurs sels d'addition non toxiques.

5. Utilisation thérapeutique caractérisée en ce que l'on utilise une substance choisie parmi l'ensemble comprenant :
(i) la 1-(4-acétylamino-3,5-dichlorophényl)-2-diméthylaminopropanone, (ii) la 1-(4-amino-3,5-dichlorophényl)-2-diméthylaminopropanone et (iii) leurs sels d'addition non-toxiques, pour l'obtention d'un médicament antidépresseur du SNC destiné à une utilisation en thérapeutique humaine vis-à-vis des dépressions et des états dépressifs.

6. Utilisation thérapeutique caractérisée en ce que l'on utilise une substance choisie parmi l'ensemble comprenant la 1-(4-acétylamino-3,5-dichlorophényl)-2-diméthylaminopropanone et ses sels d'addition non toxiques, pour l'obtention d'un médicament immuno-stimulant destiné à une utilisation en thérapeutique humaine dans le cas où une immuno-stimulation est requise.

7. Procédé de préparation d'un composé de formule I selon la revendication 1, ledit procédé étant caractérisé en ce qu'il consiste :
1°) à faire réagir 1 mole de 1-(4-aminophényl)-2-diméthylaminopropanone avec au moins 2 moles de N-chlorosuccinimide, à une température comprise entre 5 et 20°C pendant au moins 4 h, pour obtenir le composé de formule I où R = H ; et,
2°) si nécessaire, à soumettre ledit composé de formule I où R = H, à une réaction d'acétylation, pour obtenir le composé de formule I où R = $CH_3CO$.

8. Procédé suivant la revendication 7, caractérisé en ce que au stade 1°) l'on introduit par fraction le chlorosuccinimide dans une solution de 1-(4-aminophényl)-2-diméthylaminopropanone dans un solvant approprié, à une température de 10°C, puis poursuit la réaction après introduction complète du chlorosuccinimide, à une température de 15-20°C pendant 8-12 h.

9. Procédé suivant la revendication 7, caractérisé en ce que, au stade 2°) on fait réagir 1 mole de 1-(4-amino-3,5-dichlorophényl)-2-diméthylaminopropanone avec au moins 3 moles de chlorure d'acétyle, dans de l'acide acétique, à une température de 15-20°C pendant 8-12 h.

**Revendications pour les Etats contractants suivants : ES, GR**

1. Procédé de préparation d'un composé chloré de 1-(4-aminophényl)-2-dimethylaminopropanone choisi parmi :
(a) les composés répondant à la formule générale

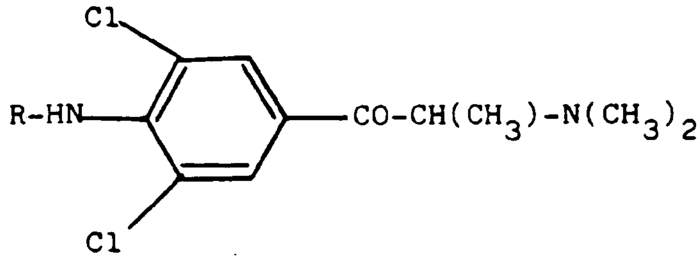

(I)

où R est H ou $CH_3CO$, et

(b) leurs sels d'addition.

ledit procédé étant caractérisé en ce qu'il comprend :

1°) la réaction de 1 mole de 1-(4-aminophenyl)-2-diméthylaminopropanone avec au moins 2 moles de N-chlorosuccinimide, à une température comprise entre 5 et 20°C pendant au moins 4 h pour obtenir le composé de formule I où R = H ; et,

2°) si nécessaire, la soumission dudit composé de formule I où R = H, à une réaction d'acétylation, pour obtenir le composé de formule I où R = $CH_3CO$.

2. Procédé suivant la revendication 1, caractérisé en ce que au stade 1°) l'on introduit par fraction le chlorosuccinimide dans une solution de 1-(4-aminophényl)-2-diméthylaminopropanone dans un solvant approprié, à une température de 10°C, puis poursuit la réaction après introduction complète du chlorosuccinimide, à une température de 15-20°C pendant 8-12 h.

3. Procédé suivant la revendication 1, caractérisé en ce que, au stade 2°) on fait réagir 1 mole de 1-(4-amino-3,5-dichlorophényl)-2-diméthylaminopropanone avec au moins 3 moles de chlorure d'acétyle, dans de l'acide acétique, à une température de 15-20°C pendant 8-12 h.

## Patentansprüche

### Patentansprüche für folgende Vertragsstaaten : AT, BE, CH, FR, GB, DE, IT, LI, LU, NL, SE

1. Derivat des 1-(4-Aminophenyl)-2-dimethylaminopropanons, dadurch gekennzeichnet, daß es aus der Gruppe bestehend aus:

(a) den Verbindungen, die der allgemeinen Formel

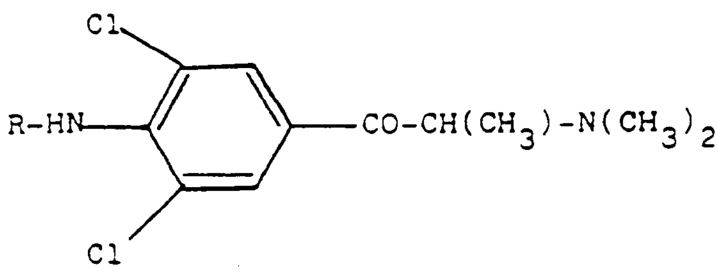

(I)

worin R H oder $CH_3CO$ ist, entsprechen und

(b) ihren Additionssalzen ausgewählt ist.

2. 1-(4-Acetylamino-3,5-dichlorphenyl)-2-dimethylaminopropanon und seine Additionssalze.

3. 1-(4-Amino-3,5-dichlorphenyl)-2-dimethylaminopropanon und seine Additionssalze.

4. Therapeutisches Präparat, dadurch gekennzeichnet, daß es in Verbindung mit einem physiologisch annehmbaren Excipiens, mindestens eine Verbindung, ausgewählt aus den Verbindungen der Formel I nach Anspruch 1, und ihren nicht-toxischen Additionssalzen, einschließt.

5. Therapeutische Verwendung, dadurch gekennzeichnet, daß eine Substanz, ausgewählt aus der Gruppe

umfassend: (i) 1-(4-Acetylamino-3,5-dichlorphenyl)-2-dimethylaminopropanon, (ii) 1-(4-Amino-3,5-dichlorphenyl)-2-dimethylaminopropanon und (iii) ihre nicht-toxischen Additionssalze verwendet wird, um ein antidepressives Medikament mit Wirkung auf das Zentralnervensystem zu erhalten, das für eine Verwendung bei der Human-Therapeutik gegen Degressionen und degressive Zustände bestimmt ist.

**6.** Therapeutische Verwendung, dadurch gekennzeichnet, daß eine Substanz, ausgewählt aus der Gruppe umfassend 1- (4-Acetylamino-3,5-dichlorphenyl)-2-dimethylaminopropanon und seine nicht-toxischen Additionssalze, verwendet wird, um ein Immunstimulans zu erhalten, das zu einer Verwendung bei der Human-Therapeutik, bei der ein Immunstimulans erforderlich ist, bestimmt ist.

**7.** Verfahren zur Herstellung einer Verbindung der Formel I nach Anspruch 1, wobei das genannte Verfahren dadurch gekennzeichnet ist, daß es umfaßt:

1.) die Umsetzung von 1 Mol 1-(4-Aminophenyl)-2-dimethylaminopropanon mit mindestens 2 Mol N-Chlorsuccinimid, bei einer Temperatur zwischen 5 und einschließlich 20° C mindestens 4 Stunden lang, um die Verbindung der Formel I, worin R = H, zu erhalten.

2.) falls erforderlich, daß die genannte Verbindung der Formel I, worin R = H, einer Acetylierungsreaktion unterworfen wird, um die Verbindung der Formel I, worin R = $CH_3CO$, zu erhalten.

**8.** Verfahren nach Anspruch 7, dadurch gekennzeichnet daß in Stufe 1.) das Chlorsuccinimid schrittweise in eine Lösung aus 1-(4-Aminophenyl)-2-dimethylaminopropanon in einem geeigneten Lösungsmittel, bei einer Temperatur von 10 ° C eingetragen wird, daß dann, nach dem vollständigen Eintrag des Chlorsuccinimids, die Reaktion bei einer Temperatur von 15-20° C während 8-12 h weitergeführt wird.

**9.** Verfahren nach Anspruch 7, dadurch gekennzeichnet, daß in Stufe 2.) 1 Mol 1-(4-Amino-3,5-dichlorphenyl)-2-dimethylaminopropanon in Essigsäure mit mindestens 3 Mol Acetylchlorid bei einer Temperatur von 15-20°C während 8-12 h umgesetzt wird.

**Patentansprüche für folgende Vertragsstaaten : ES, GR**

**1.** Verfahren zur Herstellung einer chlorierten Verbindung des 1-(4-Aminophenyl)-2-dimethylaminopropanons, ausgewählt aus:

(a) den Verbindungen, die der allgemeinen Formel

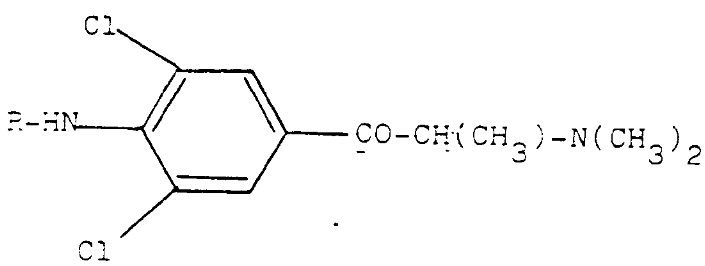

(I)

worin R H oder $CH_3CO$ ist, entsprechen und

(b) ihren Additionssalzen,

wobei das genannte Verfahren dadurch gekennzeichnet ist, daß es umfaßt:

1.) die Umsetzung von 1 Mol 1-(4-Aminophenyl)-2-dimethylaminopropanon mit mindestens 2 Mol N-Chlorsuccinimid während mindestens 4 h bei einer Temperatur zwischen 5 und einschließlich 20° C, um die Verbindung der Formel I, worin R = H, zu erhalten.

2.) falls erforderlich, daß die genannte Verbindung der Formel I, worin R = H, einer Acetylierungsreaktion unterworfen wird, um die Verbindung der Formel I, worin R = $CH_3CO$, zu erhalten.

**2.** Verfahren nach Anspruch 1, dadurch gekennzeichnet, daß in der Stufe 1.) das Chlorsuccinimid stufenweise bei einer Temperatur von 10° C in eine Lösung aus 1-(4-Aminoghenyl)-2-dimethylaminopropanon in einem geeigneten Lösungsmittel eingetragen wird, daß dann, nach dem vollständigen Eintrag des Chlorsuccinimids, die Reaktion bei einer Temperatur von 15-20° C während 8-12 h weitergeführt wird.

**3.** Verfahren nach Anspruch 1, dadurch gekennzeichnet, daß in der Stufe 2.) 1 Mol 1-(4-Amino-3,5-Dichlorphenyl)-2-dimethylaminopropanon während 8-12 h bei einer Temperatur von 15-20° C mit mindestens 3 Mol Acetylchlorid in Essigsäure umgesetzt wird.

## Claims

### Claims for the following Contracting States : AT, BE, CH, DE, FR, GB, IT, LI, LU, NL, SE

1. A 1-(4-aminophenyl)-2-dimethylaminopropanone derivative which is selected from the group consisting of:
(a) the compounds having the general formula

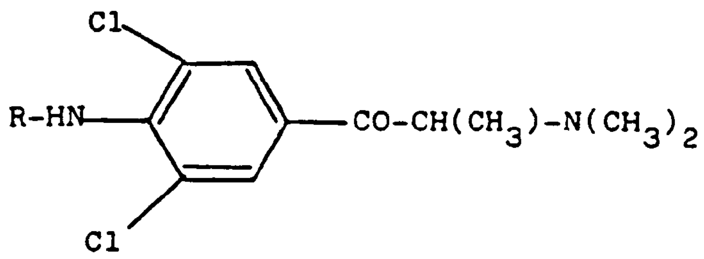

(I)

in which R is H or $CH_3CO$, and
(b) addition salts thereof.

2. 1-(4-Acetylamino-3,5-dichlorophenyl)-2-dimethylaminopropanone and addition salts thereof.

3. 1-(4-Amino-3,5-dichlorophenyl)-2-dimethylaminopropanone and addition salts thereof.

4. A therapeutic composition which contains, in association with a physiologically acceptable excipient, at least one compound selected from the compounds of formula I according to claim 1 and non-toxic addition salts thereof.

5. A therapeutical use which comprises using a substance selected from the group consisting of:
(i) 1-(4-acetylamino-3,5-dichlorophenyl)-2-dimethglaminopropanone, (ii) 1-(4-amino-3,5-dichlorophenyl)-2-dimethylaminopropanone and (iii) non-toxic addition salts thereof,
for the preparation of an antidepressant for the CNS which is to be used in human therapy for the treatment of depressions and depressive states.

6. A therapeutical use which comprises using a substance selected from the group consisting of 1-(4-acetylamino-3, 5-dichlorophenyl)-2-dimethylaminopropanone and non-toxic addition salts thereof, for the preparation of an immunostimulant which is to be used in human therapy in the case where immunostimulation is required.

7. A method for the preparation of a compound of formula I according to claim 1, the said method consisting in:
1°) reacting 1 mol of 1-(4-aminophenyl)-2-dimethylaminopropanone with at least 2 mol of N-chlorosuccinimide, at a temperature of between 5 and 20°C, for at least 4 h, to give the compound of formula I in which R = H; and
2°) if necessary, subjecting the said compound of formula I in which R = H to an acetylation reaction to give the compound of formula I in which R = $CH_3CO$.

8. The method according to claim 7, wherein, in stage 1°), the chlorosuccinimide is introduced in portions into a solution of 1-(4-aminophenyl)-2-dimethylaminopropanone in an appropriate solvent, at a temperature of 10°C, and the reaction is then continued, after introduction of the chlorosuccinimide is complete, at a temperature of 15-20°C for 8-12 h.

9. The method according to claim 7, wherein, in stage 2°), 1 mol of 1-(4-amino-3,5-dichlorophenyl)-2-dimethylaminopropanone is reacted with at least 3 mol of acetyl chloride in acetic acid at a temperature of 15-20°C for 8-12 h.

**Claims for the following Contracting States : ES, GR**

1. A method for the preparation of a chloro compound of 1-(4-aminophenyl)-2-dimethylaminopropanone selected from :
   (a) the compounds having the general formula

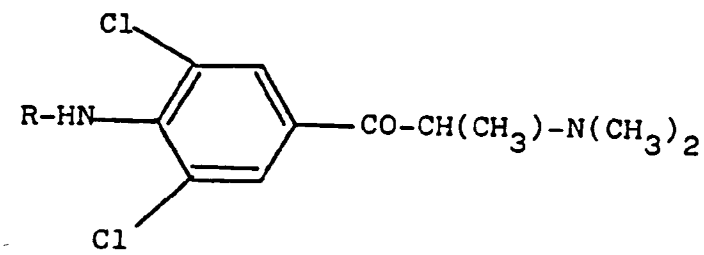

(I)

   in which R is H or $CH_3CO$, and
   (b) addition salts thereof,
   said method consisting in :
   1°) reacting 1 mol of 1-(4-aminophenyl)-2-dimethylaminopropanone with at least 2 mol of N-chlorosuccinimide, at a temperature of between 5 and 20°C, for at least 4 h, to give the compound of formula I in which R = H ; and
   2°) if necessary, subjecting the said compound of formula I in which R = H to an acetylation reaction to give the compound of formula I in which R = $CH_3CO$.

2. The method according to claim 1, wherein, in stage 1°) the chlorosuccinimide is introduced in portions into a solution of 1-(4-aminophenyl)-2-dimethylaminopropanone in an appropriate solvent, at a temperature of 10°C, and the reaction is then continued, after introduction of the chlorosuccinimide is complete, at a temperature of 15-20°C for 8-12 h.

3. The method according to claim 1, wherein in stage 2°), 1 mol of 1-(4-amino-3,5-dichlorophenyl)-2-dimethylaminopropanone is reacted with at least 3 mol of acetyl chloride in acetic acid at a temperature of 15-20°C for 8-12 h.